Europäisches Patentamt

European Patent Office

Office européen des brevets

(11) Veröffentlichungsnummer: **0 111 078**
**B1**

(12) # EUROPÄISCHE PATENTSCHRIFT

(45) Veröffentlichungstag der Patentschrift:
12.08.87

(51) Int. Cl.⁴: **A 23 L 1/00**, A 23 L 3/26,
A 61 L 2/02

(21) Anmeldenummer: 83109684.7

(22) Anmeldetag: 28.09.83

(54) *Verfahren und Vorrichtung zur Behandlung von für den menschlichen Kontakt allergisierenden Produkten und Gegenständen.*

(30) Priorität: 05.11.82 DE 3240967
09.03.83 DE 3308256
05.11.82 DE 8231066 U

(43) Veröffentlichungstag der Anmeldung:
20.06.84 Patentblatt 84/25

(45) Bekanntmachung des Hinweises auf die Patenterteilung:
12.08.87 Patentblatt 87/33

(84) Benannte Vertragsstaaten:
AT BE CH DE FR GB IT LI LU NL SE

(56) Entgegenhaltungen:
AU-B-39 033
DE-A-1 467 785
DE-A-3 035 914

(73) Patentinhaber: Pose, Wolfgang, Dr., Goernestrasse
9, D-2000 Hamburg 20 (DE)

(72) Erfinder: Pose, Wolfgang, Dr., Goernestrasse 9,
D-2000 Hamburg 20 (DE)

(74) Vertreter: Patentanwälte Dipl.- Ing. J. Richter
Dipl.- Ing. F. Werdermann, Neuer Wall 10, D-2000
Hamburg 36 (DE)

## Beschreibung

Die Erfindung betrifft ein Verfahren und eine Vorrichtung zur Behandlung von für den menschlichen Kontakt allergisierenden Produkten und Gegenständen, die Genußmittel, Getränke, Möbel, Bauelemente, (Spanplatten aller Art), Wäsche, Windeln, Fußbodenbeläge aller Art, zahnärztliche Werkstoffe aller Art, Textilien, u.dgl., wie Pestizide, Schwermetalle, Lösungsmittel, Weichmacher, Säuren, Herbizide, Kunstdünger, Konservierungsmittel, Wachstumshemmer, Formaldehyde, Medikamentenrückstände u.dgl. enthalten.

Es ist bekannt, daß die für den menschlichen Verzehr vorgesehenen Lebensmittel oftmals durch Umweltgifte belastet sind. Insbesondere bei einem Freilandanbau von pflanzlichen Lebensmitteln sind diese oft durch Pflanzenschutzmittel, wie Insektizide, Fungizide oder Pestizide, belastet, da diese Pflanzenschutzmittel den Lebensmitteln auch nach der Aberntung noch anhaften und für eine menschlichen Verzehr ungeeignet sind, da sie sich für den Menschen als Giftstoffe erweisen können.

Außerdem hat es sich gezeigt, daß sowohl pflanzliche als auch tierische Lebensmittel wie auch Trinkwasser durch im Wasser oder in der Luft enthaltene Giftstoffe, wie Schwermetalle, Lösungsmittel, Weichmacher, Säuren u.dgl. verseucht sind.

Darüber hinaus ist es bekannt, daß auch im Umgebungsbereich des Menschen verwendete Gegenstände, wie Möbel, Fertigbauelemente, Fußbodenbeläge u.dgl. oftmals aufgrund der verwendeten Materialien oder durch die Herstellungsverfahren Stoffe beinhalten, die auf den Menschen eine toxische oder allergisierende Wirkung haben können. Darüber hinaus ist bekannt geworden, daß auch Produkte, die für den direkten Kontakt mit menschlichen Körperteilen vorgesehen sind, wie beispielsweise zahnärztliche Werkstoffe aller Art, Textilien, Windeln u.dgl., material- oder herstellungsbedingt eine toxisch-allergisierende Wirkung auf den Menschen ausüben können. Hierbei kann davon ausgegangen werden, daß die toxisch-allergisierenden Wirkungen beim Menschen auf die in den Gegenständen und Produkten enthaltenen Giftstoffe zurückzuführen sind.

Nach der DE-A-3 035 914 ist ein Verfahren zum Frischhalten von Lebensmitteln, insbesondere Frischgemüse und Frischobst in einem Kühlraum bekannt, nach dem die Lebensmittel bei Einwirkung eines künstlich erzeugten magnetischen Feldes ausgesetzt werden. Die für dieses Verfahren verwendete Vorrichtung umfaßt Mittel zur Erzeugung des magnetischen Feldes, wie Elektromagneten oder einen Permanentmagneten.

Ein Verfahren und eine Vorrichtung, um organische Substanzen länger frisch zu halten oder schneller in Gärung zu versetzen, sind der DE-A-1 467 785 zu entnehmen. Das Verfahren besteht darin, daß die frisch zu haltende Substanz ganz oder teilweise in einem magnetischen Kraftfeld gelagert wird. Die hierzu vorgesehene Vorrichtung ist derart ausgebildet, daß die frisch zu haltende Substanz in einem elektrisch neutralen Behälter aufbewahrt wird, der von Permanent- oder Elektromagneten umgeben ist. Hiernach wird die frisch zu haltende Substanz in einem magnetischen Kraftfeld gelagert, für dessen Erzeugung Permanent- oder Elektromagneten vorgesehen sind, die so angeordnet sind, daß diese die frisch zu haltende Substanz umgeben. Eine Anordnung der Permanent- oder Elektromagneten in einem Abstand oberhalb oder unterhalb der frisch zu haltenden Substanz ist nicht vorgesehen. Die zur Erzeugung des magnetischen Feldes verwendete Magnetfelderzeugungseinrichtung kann dabei auch dreh- oder schwenkbar angeordnet sein. Eine oszillierende Bewegung ist nicht vorgesehen.

Auch die AU-B-39 033 befaßt sich mit dem Frischhalten von Lebensmitteln unter Verwendunbg eines Magnetfeldes.

Keines der bekannten Verfahren befaßt sich mit der Behandlung von für den menschlichen Kontakt allergisierenden Produkten und Gegenständen, sondern ausschließlich mit der Konservierung von Lebensmitteln unter Verwendung magnetischer Felder.

Die Erfindung löst die Aufgabe, ein Verfahren zur Behandlung von für den menschlichen Kontakt allergisierenden Produkten und Gegenständen und eine Vorrichtung zur Durchführung des Verfahrens zu schaffen.

Zur Lösung dieser Aufgabe sieht die Erfindung ein Verfahren zur Behandlung von für den menschlichen Kontakt allergisierenden Produkten und Gegenständen, wie Nahrungsmittel, Genußmittel, Getränke, Möbel, Bauelemente (Spanplatten aller Art), Wäsche, Windeln, Fußbodenbeläge aller Art, zahnärztliche Werkstoffe aller Art, Textilien u.dgl., die Pestizide, Schwermetalle, Lösungsmittel, Weichmacher, Säuren, Herbizide, Kunstdünger, Konservierungsmittel, Wachstumshemmer, Formaldehyde, Medikamentenrückstände u.dgl. enthalten vor, nach dem erfindungsgemäß die zu behandelnden Produkte und Gegenstände mit einem Magnetfeld bei gleichzeitiger oszillierender Relativbewegung zwischen der Magnetfelderzeugungseinrichtung und den Produkten und Gegenständen beaufschlagt werden.

Zur Lösung dieser Aufgabe sieht die Erfindung ferner eine Vorrichtung zur Behandlung von für den menschlichen Kontakt allergisierenden Produkten und Gegenständen unter Verwendung einer in einem Gehäuse angeordneten, bewegbaren Magnetfelderzeugungseinrichtung und einer Produktaufnahmefläche vor, die erfindungsgemäß in der Weise ausgebildet ist, daß die Produktaufnahmefläche in einem Abstand zur Magnetfelderzeugungseinrichtung angeordnet ist und daß die Magnetfelderzeugungseinrichtung in eine oszillierende Bewegung versetzbar ist, wobei die Magnetfelderzeugungseinrichtung aus einem Elektromagneten und einer Magnetsteuereinrichtung oder aus einem Permanentmagneten und einer Permanentmagnetabschirmeinrichtung besteht.

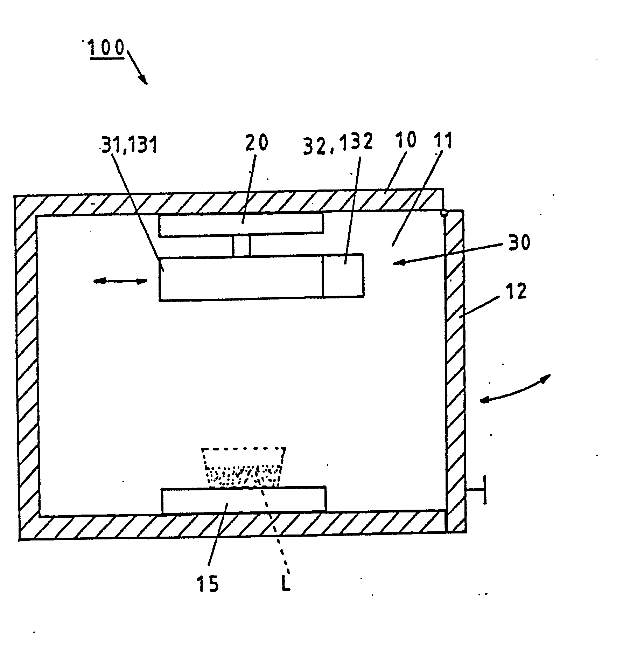

In der Zeichnung ist der Gegenstand der Erfindung beispielsweise dargestellt, und zwar zeigt die Zeichnung ein Ausführungsbeispiel einer Vorrichtung zur Behandlung von für den menschlichen Kontakt allergisierenden Produkten und Gegenständen in einem senkrechten Schnitt in einer schematischen Darstellung.

In der Zeichnung ist die Vorrichtung mit 100 bezeichnet, die aus einem Gehäuse 10 mit einer in dessen Innenraum 11 angeordneten Magnetfelderzeugungseinrichtung 30 besteht. Die Magnetfelderzeugungseinrichtung 30 ist so mit einer am Gehäuse 10 befestigten Antriebseinrichtung 20 verbunden, daß die Magnetfelderzeugungseinrichtung 30 in eine durch einen Doppelpfeil gekennzeichnete oszillierende Bewegung versetzbar ist. Die Magnetfelderzeugungseinrichtung 30 besteht aus einem Elektromagneten 31 und einer entsprechenden Magnetsteuerungseinrichtung 32, mit der die Stärke des durch den Elektromagneten 31 erzeugten Magnetfeldes regel- und steuerbar ist.

Der Innenraum 11 des Gehäuses 10 ist über eine Gehäuseklappe 12, die am Gehäuse 10 schwenkbar angelenkt ist, eingreifbar und weist eine am Gehäuse 10 angebrachte Produktaufnahmefläche 15 auf, die so angeordnet ist, daß sie in geeigneter Weise im Bereich des von der Magnetfelderzeugungseinrichtung 30 erzeugten Magnetfeldes liegt.

Zur Behandlung von Lebensmitteln werden diese, wie das in der Zeichnung gestrichelt eingezeichnet und mit L bezeichnete Lebensmittel, auf die Produktaufnahmefläche 15 aufgebracht. Anschließend werden die Magnetfelderzeugungseinrichtung 30 und die Antriebseinrichtung 20 eingeschaltet, so daß im Gehäuseinnenraum 11 ein entsprechendes Magnetfeld ausgebildet wird, das aufgrund der durch die Antriebseinrichtung 20 erzeugten oszillierenden Bewegung der Magnetfelderzeugungseinrichtung 30 eine räumliche Bewegung durchführt. Nachdem das Lebensmittel L während einer entsprechenden, vom jeweiligen Lebensmittel und den in ihm enthaltenen Rückständen abhängigen Zeitspanne dem bewegten Magnetfeld ausgesetzt war, kann nach Abschaltung der Antriebseinrichtung 20 und der Magnetfelderzeugungeinrichtung 30 das nunmehr neutralisierte Lebensmittel 11 der Vorrichtung 100 entnommen werden.

Abweichungen in der Ausgestaltung des Gehäuses 10 und der Anordnung der im Gehäuse angebrachten Magnetfelderzeugungseinrichtung 30 sind möglich, ebenso auch eine andersartige, beispielsweise aus einem Permanentmagneten, bestehende Magnetfelderzeugungseinrichtung 30, wobei jedoch bei allen Ausführungsformen der Vorrichtung 100 eine Bewegung des durch die Magnetfelderzeugungseinrichtung 30 erzeugten Magnetfeldes gegeben sein muß. Außerdem kann die Magnetsteuereinrichtung 32 bzw. die Permanentmagnetabschirmeinrichtung 132 so ausgebildet sein, daß eine automatische Behandlung, d.h. die Durchführung der Beaufschlagung der zu behandelnden Produkte mit einem Magnetfeld, vollautomatisch abläuft.

Darüber hinaus ist es auch möglich, das beschriebene Verfahren in Abgasreinigungseinrichtungen, wie Kfz-Abgasfiltereinrichtungen oder Industrieabgasfiltereinrichtungen, anzuwenden, wobei sowohl die Filtermedien behandelt werden können als auch unter Verwendung einer geeigneten, in der Zeichnung nicht dargestellten Vorrichtung eine Behandlung des Abgases direkt durchgeführt werden kann. Eine Anwendung bei Trinkwasseraufbereitungsanlagen bzw. Abwasserreinigungsanlagen ist ebenfalls möglich.

**Patentansprüche**

1. Verfahren zur Behandlung von für den menschlichen Kontakt allergisierenden Produkten und Gegenständen, wie Nahrungsmittel, Genußmittel, Getränke, Möbel, Bauelemente (Spanplatten aller Art), Wäsche, Windeln, Fußbodenbeläge aller Art, zahnärztliche Werkstoffe aller Art, Textilien u.dgl., die Pestizide, Schwermetalle, Lösungsmittel, Weichmacher, Säuren, Herbizide, Kunstdünger, Konservierungsmittel, Wachstumshemmer, Formaldehyde, Medikamentenrückstände u.dgl., enthalten, dadurch gekennzeichnet, daß die zu behandelnden Produkte und Gegenstände mit einem Magnetfeld bei gleichzeitiger oszillierender Relativbewegung zwischen der Magnetfelderzeugungseinrichtung und den Produkten und Gegenständen beaufschlagt werden.

2. Vorrichtung zur Durchführung des Verfahrens nach Anspruch 1 unter Verwendung einer in einem Gehäuse (10) angeordneten, bewegbaren Magnetfelderzeugungseinrichtung (30) und einer Produktaufnahmefläche (15), dadurch gekennzeichnet, daß die Produktaufnahmefläche (15) in einem Abstand zur Magnetfelderzeugungseinrichtung (30) angeordnet ist und daß die Magnetfelderzeugungseinrichtung (30) in eine oszillierende Bewegung versetzbar ist, wobei die Magnetfelderzeugungeinrichtung (30) aus einem Elektromagneten (31) und einer Magnetsteuereinrichtung (32) oder aus einem Permanentmagneten (131) oder aus einer Permanentmagnetabschirmeinrichtung (132) besteht.

## Claims

1. Procedure for the treatment of allergising products and objects intended for human contact, like food, coffee, tea, cigarettes etc., beverages, furniture, elements (all kinds of chip boards), linen, baby napkins, all kinds of floor covering, all kinds of dental materials, textiles and similar containing pesticides, heavy metals, solvents, softeners, acids, herbicides, artificial fertilizers, preservatives, growth inhibitors, formaldehydes, medicine residues and similar, characterized in that the products and objects to be treated are admitted by a magnetic field with simulatuous oscillating relative movement between the device producing the magnetic field and the products and objects.

2. Device to carry out the procedure according to claim 1 a device for the production of a magnetic field (30) mounted in a casing (10) and a surface for receiving the product (15), characterized in that the surface for receiving the product (15) is arranged at a certain distance from the device for the production of a magnetic field (30), and that the device for the production of a magnetic field (30) is brought into an oscillationg movement, the device for the production of a magnetic field (30) comprising an electromagnet (31) and a magnet control device (32) or a pemanent magnet (131) or a permanent magnet shielding device (132).

## Revendications

1. Procédé pour le traitement de produits et d'objets causant des allergies, tels que des aliments, stimulants, boissons, meubles, éléments de construction (panneaux de particules de tout type), linge, couches, revêtements de sol de tout type, matériaux dentaires de tout type, textiles ou équivalents, qui contiennent des pesticides, métaux lourds, solvants, adoucissants, acides, herbicides, engrais chimiques, agents conservateurs, agents empêchant la croissance, aldéhydes formiques, résidus de médicament ou équivalents, caractérisé en ce que l'on soumet les produits et objets à traiter à un champ magétique, un mouvement relatif oscillant étant simultanément réalisé entre le dispositif de production de champs magétiques et les produits et objets.

2. Dispositif pour la réalisation du procédé selon la revendication 1 qui utilise un dispositif (30) de production de champs magétiques mobile placé dans un bâti (10) et une surface de logement du produit (15), caractérisé en ce que la surface de logement du produit (15) est placée à une certaine distance du dispositif (30) de production de champs magétiques et que le dispositif (30) de production de champs magnétiques peut être déplacé par un mouvement oscillant, le dispositif (30) de production de champs magnétiques étant composé d'un électro-aimant (31) et d'un dispositif de commande de l'aimant (32) ou d'un aimant permanent (131) ou d'un dispositif de blindage pour aimant permanent (132).